(19) Europäisches Patentamt
European Patent Office
Office européen des brevets

(11) Publication number: 0 022 288 A2

(12) # EUROPEAN PATENT APPLICATION

(21) Application number: 80200590.0

(22) Date of filing: 20.06.80

(51) Int. Cl.³: A 01 N 43/36
C 07 D 209/52

(30) Priority: 06.07.79 GB 7923714

(43) Date of publication of application:
14.01.81 Bulletin 81/2

(84) Designated Contracting States:
CH DE FR GB IT LI NL

(71) Applicant: SHELL INTERNATIONALE RESEARCH MAATSCHAPPIJ B.V.
Carel van Bylandtlaan 30
NL-2596 HR DEN HAAG(NL)

(72) Inventor: Haddock, Ernest
187 Queenborough Road
Sheerness Kent(GB)

(72) Inventor: Hopwood, William John
24 Blandford Gardens
Sittingbourne Kent(GB)

(74) Representative: Keuzenkamp, Abraham et al,
c/o Shell Internationale Research Maatschappij B.V. P.O. Box 302
NL-2501 CH 's-Gravenhage(NL)

(54) **Method of sterilizing male anthers in plants, compositions and compounds suitable for use in such a method, and method of producing F1 hybrid seed.**

(57) A method of sterilizing the male anthers of a plant, which comprises applying to the plant a pyrrolidine derivative which is a compound of the general formula I or a salt and or an ester, N-alkylamide, N,N-dialkylamide, the amide or hydrazide thereof:

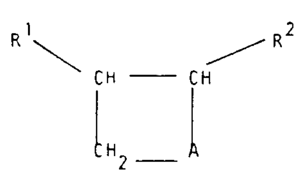

in which A represents -NH-CH-$CO_2H$ or -N = C-$CO_2H$, and each of $R^1$ and $R^2$, which may be the same or different, represents, a halogen atom, a hydroxy group or an alkyl or alkoxy group having from 1 to 6 carbon atoms and optionally substituted by one or more of the same or different halogen atoms, or $R^1$ and $R^2$ together represent an epoxy group, a group of the formula -O-$CH_2$-O-, or a di- or tri-methylene group.

EP 0 022 288 A2

# Method of sterilizing male anthers in plants, compositions and compounds suitable for use in such a method, and method of producing $F_1$ hybrid seed

This invention relates to a method of sterilising male anthers in plants, to compositions and compounds for use in such a method, and to $F_1$ hybrid seeds produced by such a method.

To obtain $F_1$ hybrid seeds, which have many advantages over non-hybrid seeds, seed-breeders cross-pollinate carefully selected parent plants. In the case of plants, for example small-grain cereal plants, which have hermaphroditic flowers and normally self-pollinate, this is achieved by removing the male anthers from each of the flowers by hand, an operation which is extremely time-consuming and requires highly-skilled workers. Much research is being carried out into treatments with chemicals by which this same result can be achieved.

The present invention provides a method of sterilising the male anthers of a plant, which comprises applying to the plant a pyrrolidine derivative which is a compound of the general formula I or a salt and/or an ester, N-alkylamide, N,N-dialkylamide, the amide or hydrazide thereof:

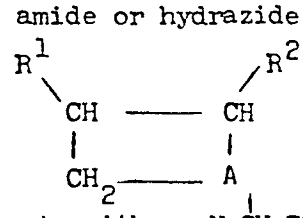

(I)

in which A represents either $-\underset{H}{N}-\overset{|}{C}H-CO_2H$ or $-N=\overset{|}{C}-CO_2H$, and each of $R^1$ and $R^2$, which may be the same or different, represents a halogen atom, a hydroxy group or an alkyl or alkoxy group having from 1 to 6 carbon atoms and optionally substituted

BAD ORIGINAL

by one or more of the same or different halogen atoms, or $R^1$ and $R^2$ together represent an epoxy group, a group of formula $-O-CH_2-O-$ or a di- or tri-methylene group.

Preferably in the pyrrolidine derivative each of $R^1$ and $R^2$, which may be the same or different, represents an alkyl or alkoxy group having 1 or 2 carbon atoms or a chlorine or bromine atom, or $R^1$ and $R^2$ together represent an epoxy group or a di- or tri-methylene group. More preferably, each of $R^1$ and $R^2$, which may be the same or different, represents a methyl or methoxy group or a chlorine atom, or $R^1$ and $R^2$ together represent a dimethylene group.

Preferably A represents the group $-NH-\overset{|}{CH}-CO_2H$.

The pyrrolidine derivative may for example be the free acid of the general formula I, a hydrohalide or an alkali metal salt thereof, the amide or hydrazide thereof, an alkyl ester having from 1 to 10, for example 1 to 6, carbon atoms in the alkyl group, or a hydrohalide of said ester, amide or hydrazide.

The pyrrolidine derivatives used in the process of the invention may exist in the form of various isomers. For example, the groups $R^1$ and $R^2$ may be cis or trans to each other, and in the saturated ring compounds the 2-carbonyl group may be cis or trans relative to $R^1$ and/or $R^2$. Further, possibilities for optical isomerism exist. Preferably the process according to the invention is carried out using a pyrrolidine derivative at least some of which is present in its 3,4-cis isomeric form having the general formula

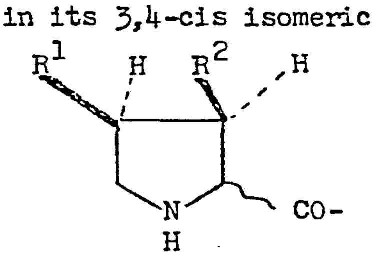 (Ia) or 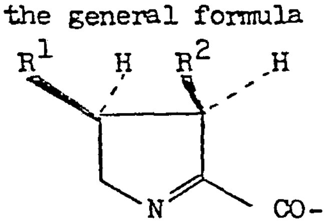 (Ib)

Preferably the pyrrolidine derivative is present predominantly in said cis-isomeric form (i.e. there being a greater amount of 3,4-cis isomer present than 3,4-trans isomer), and an especially preferred embodiment of the invention uses a pyrrolidine derivative substantially all of which is present in 3,4-cis isomeric form. Certain pyrrolidine derivatives can of course only exist as a 3,4-

cis isomer: for example those compounds in which $R^1$ and $R^2$ together with the interjacent carbon atoms form a 3 or 4 membered ring are geometrically constrained in the 3,4-cis form.

The process according to the invention produces plants in which male sterility has been produced without substantial effect on female fertility. Preferably the process is applied to cereal plants, for example maize and sorghum, especially to small-grain cereal plants such as wheat and barley, preferably when the plant is at a stage of growth between late tillering and emergence of the ear. The active compound is suitably applied at a dosage of from 0.05 to 2 kg/ha, preferably 0.25 to 1 kg/ha. The present invention also provides a method of producing $F_1$ hybrid seed, which includes cross-pollinating a plant which has been treated by a process according to the invention with a second plant of a different strain.

The pyrrolidine derivative is suitably formulated as a pollen-suppressing composition for use in the process of the invention. The invention therefore also provides a pollen-suppressing composition which comprises a compound of the general formula I or a salt and/or an ester, N-alkylamide, N,N-dialkylamide, the amide or hydrazide thereof together with a suitable carrier.

The invention also provides a compound of the general formula I or a salt and/or an ester, N-alkylamide, N,N-dialkylamide, the amide or hydrazide thereof, in which $R^1$ and $R^2$ have the meanings given above with the proviso that, if the compound is the free acid of the general formula I in which A represents the group $-NH-\overset{|}{C}H-CO_2H$ and $R^1$ and $R^2$ both represent methyl groups, then these methyl groups are in cis relationship to each other. Suitably if the compound is a free acid of the general formula I, then at least one of $R^1$ and $R^2$ is other than a methyl group.

The invention also provides a process for the preparation of a novel compound according to the invention, which comprises either

(a) converting a compound of the general formula

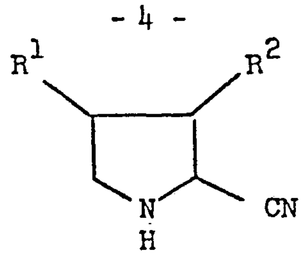

(II)

in which $R^1$ and $R^2$ have the meanings given for the general formula I, into a compound according to the invention, or

(b) hydrolysing, decarboxylating and reducing a diester of a diacid of the general formula

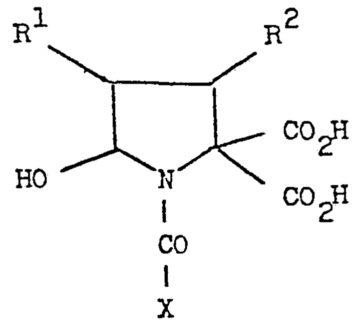

(III)

in which $R^1$ and $R^2$ have the meanings given for the general formula and X represents an alkyl, preferably a methyl, group; and if desired,

(c) converting at least part of the compound according to the invention obtained from (a) or (b) into any other compound according to the invention.

In process variant (a), the cyanide of formula II may be converted into a saturated ring compound according to the invention in many ways. For example, acid hydrolysis, for example using a concentrated mineral acid, e.g. hydrochloric acid, leads to the free acid of the formula I or an acid addition salt thereof, while alkaline hydrolysis leads to an alkali metal salt thereof. Reaction with an alcohol leads to an ester, for example reaction with ethanol in the presence of thionyl chloride, or dry hydrogen chloride, leads to the ethyl ester of a compound of the general formula I.

The compound of the general formula II may be prepared by the addition of hydrogen cyanide or an alkali metal bisulphite followed by an alkali metal cyanide, to a compound of the general formula

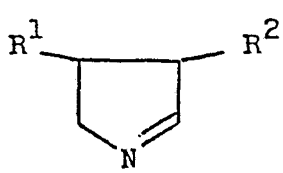

(IV)

which may exist in trimeric form. The compound of the general formula IV may be prepared by the following sequence of reactions:

(i) reaction of an acid of the formula

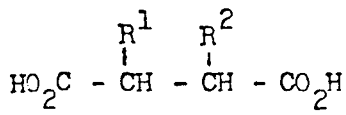

(V)

or the anhydride thereof, with benzylamine, to produce a compound of the general formula

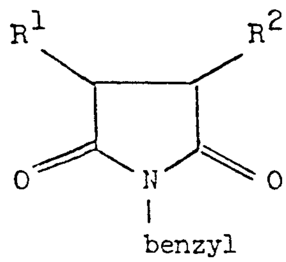

(VI)

If $R^1$ and $R^2$ together with the interjacent carbon atoms represent a ring as defined for formula I, the two carboxy groups in the acid of the formula V should be in the cis position to permit cyclisation. This reaction is suitably carried out in the presence or absence of a solvent at a temperature in the range of from 150 to 200°C, while distilling off the water formed.

(ii) Selective reduction of the carbonyl groups of the compound of the formula VI using complex aluminium hydride reducing agent, for example lithium aluminium hydride or sodium bis(2-methoxyethoxy)aluminium dihydride, to produce a compound of the general formula

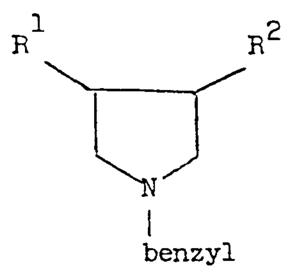

(VII)

(iii) Hydrogenolysing the N-benzyl bond of a compound of the general formula VII using hydrogen and a catalyst, preferably a palladium catalyst, to produce a compound of the general formula

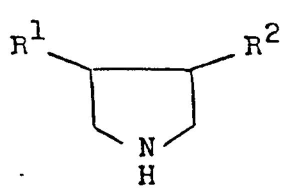

BAD ORIGINAL

(VIII)

(iv) N-chlorinating or N-brominating a compound of the general formula VIII using a suitable halogenating agent, for example N-bromo- or N-chlorosuccinimide, sodium or potassium hypochlorite or tertiary butyl hypochlorite, and subsequently dehydrohalogenating the resulting compound to give the compound of the general formula IV. Suitable dehydrohalogenating agents include organic or inorganic bases, for example pyridine and alkali metal hydroxides and alkoxides. Alcoholic solutions of sodium or potassium hydroxide or ethoxide are especially suitable.

In process variant (b), conversion of a compound of the general formula III into a saturated ring compound according to the invention may be carried out in a single step by reaction with tin and concentrated hydrochloric acid. The reaction is suitably carried out under reflux in the presence of a suitable solvent, for example water.

The compound of the general formula III may be prepared by condensing a diester of a compound of the general formula

$$X-\overset{O}{\overset{\|}{C}}-NH-CH(CO_2H)_2 \qquad (IX)$$

with a compound of the general formula

$$HOC-\overset{R^1}{\overset{|}{C}}=\overset{R^2}{\overset{|}{CH}} \qquad (X)$$

in the presence of a suitable base, for example an alkali metal alkoxide.

A compound of the general formula I can be converted into another compound of the general formula I by any suitable method. For example, a free acid of the general formula I can be converted into one of its functional derivatives by methods known in the art.

A saturated ring compound of the general formula I (i.e. one in which A represents the group $-NH-\overset{|}{C}H-CO_2H$) may be converted into an unsaturated compound (i.e. one in which A represents the group $-N=\overset{|}{C}-CO_2R$) by oxidation, for example by treatment with manganese dioxide. Alternatively, a two-stage process may be employed in which the saturated ring compound is N-chlorinated or N-brominated and subsequently dehydrohalogenated, by a method directly analogous to that described in (iv) above.

A carrier in a composition according to the invention is any material with which the active ingredient is formulated to facilitate application to the plant to be treated or to facilitate

storage, transport or handling. A carrier may be a solid or a liquid, including a material which is normally gaseous but which has been compressed to form a liquid, and any of the carriers normally used in formulating agricultural compositions may be used.

Suitable solid carriers include natural and synthetic clays and silicates, for example natural silicas such as diatomaceous earths; magnesium silicates, for example talcs; magnesium aluminium silicates, for example attapulgites and vermiculites; aluminium silicates, for example kaolinites, montmorillonites and micas; calcium carbonate; calcium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; chlorinated hydrocarbons, for example carbon tetrachloride, perchloroethylene and trichloroethane. Mixtures of different liquids are often suitable.

Compositions for use in agriculture or horticulture are often formulated and transported in a concentrated form which is subsequently diluted by the user before application. The presence of small amounts of a carrier which is a surface-active agent facilitates this process of dilution. Thus preferably at least one carrier in a composition according to the invention is a surface-active agent. For example, a composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids;

the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylphenol or p-octylcresol, with ethylene oxide and/or propylene oxide; sulphates or sulponates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may, for example contain from 0.5 to 95% by weight of active ingredient, and may for example be formulated as wettable powders, dusts, granules, solutions, emulsifiable concentrates, emulsions, suspension concentrates and aerosols. Wettable powders usually contain 25, 50 and 75% w of active ingredient and usually contain, in addition to solid inert carrier, 3-10% w of a dispersing agent and, where necessary, 0-10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½-10% w of active ingredient. Granules are usually prepared to have a size between 10 and 100 BS mesh (1.676 - 0.152 mm), and may be manufactured by agglomeration or impregnation techniques. Generally, granules will contain ½-25% w active ingredient and 0-10% w of additives such as stabilisers, slow release modifiers and binding agents. Emulsifiable concentrates usually contain, in addition to a solvent and, when necessary, co-solvent, 10-50% w/v active ingredient, 2-20% w/v emulsifiers and 0-20% w/v of other additives such as stabilisers, penetrants and corrosion inhibitors. Suspension concentrates are usually compounded so

as to obtain a stable, non-sedimenting flowable product and usually contain 10-75% w active ingredient, 0.5-15% w of dispersing agents, 0.1-10% w of suspending agents such as protective colloids and thixotropic agents, 0-10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as antifreeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The following Examples illustrate the invention.

Example 1 - Preparation of 2-carboxy-3-azabicyclo [3.2.0] heptane

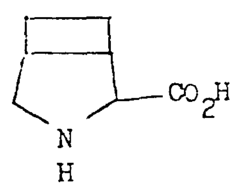

(a) 3-benzyl-3-azabicyclo [3.2.0] heptane-2,4-dione (1A)

Cis 1,2-cyclobutane carboxylic acid anhydride (12.6 g, 0.1 mol) was warmed until a melt formed, and benzylamine (10.7 g, 0.1 mol) was added dropwise under a stream of nitrogen over a period of half an hour. The mixture was heated at 170-180 °C under nitrogen until all water had been removed (about 3 hours). The residue was cooled and treated with hexane to give 17.2 g of an off-white solid, which was recrystallised from ethanol to give white crystals of 1A, melting point 51-53°C. The procedure was repeated to give a further 50 g of crystals.

| Analysis | C | H | N |
|---|---|---|---|
| Calculated for $C_{13}H_{3}NO_2$ | 72.6 | 6.0 | 6.5 |
| Found | 72.2 | 5.9 | 6.5 |

(b) 3-benzyl-3-azabicyclo[3.2.0]heptane (1B)

64.3 g (0.3 mol) of 1A were dissolved in benzene (200 ml) and the solution was added dropwise over 2 hours to sodium bis(2-methoxyethoxy)aluminium dihydride (260 ml) and then refluxed for 6 hours. The mixture was cooled and washed with 30% aqueous sodium hydroxide solution and then water (200 ml). The benzene layer was separated, dried ($MgSO_4$) and evaporated to give 45.0 g of a pale yellow oil which was distilled under reduced pressure to give 38.9 g of 1B as a colourless liquid, boiling point 65-67°C at a pressure of 0.07 mm Hg.

| Analysis | C | H | N |
|---|---|---|---|
| Calculated for $C_{13}H_{17}N$ | 83.4 | 9.1 | 7.5 |
| Found | 82.8 | 9.3 | 7.4 |

(c) 3-azabicyclo[3.2.0]heptane (1C)

7.5 g (0.04 mol) of 1B, ethanol (50 ml) and 10% palladium charcoal were mixed and hydrogenated at 4 atmospheres absolute pressure and 20°C, for 24 hours. The mixture was filtered and the filtrate evaporated using a fractionating column until all substances boiling below 100°C had been removed. The distillate was treated with concentrated hydrochloric acid (10 ml) and the mixture was evaporated to dryness to give 0.4 g of the hydrochloride salt of 1C as a white solid, melting point 194-196°C. The procedure was repeated to give a further 3.7 g of product.

Treatment of the hydrochloride salt of 1C with sodium bicarbonate solution gave an oil which was extracted with diethyl ether. The ether layer was dried ($MgSO_4$) and evaporated to give a colourless liquid which was distilled to give a 1C as a colourless liquid, boiling point 134-135°C, in 90% yield.

| Analysis | C | H | N |
|---|---|---|---|
| Calculated for $C_6H_{11}N$ | 74.2 | 11.3 | 14.4 |
| Found | 66.8 | 11.8 | 13.1 |

(d) 2-cyano-3-azabicyclo[3.2.0]heptane (1D)

N-chlorosuccinimide (23.6 g, 0.17 mol) was suspended in dry diethyl ether (500 ml) and 10.8 g (0.1 mol) of 1C was added. The mixture was stirred at 20°C for 20 hours and then filtered. The ether layer was washed with water (2 x 200 ml)

and sodium chloride solution (100 ml). The ether layer was separated, dried ($MgSO_4$) and evaporated carefully to about 100 ml. This solution was added carefully at 0°C to potassium hydroxide (5.6 g, 0.1 mol) in ethanol (5.0 ml). The resulting mixture was then stirred at 20°C for 16 hours.

The mixture was filtered, and the filtrate treated with sodium metabisulphite (9.5 g, 0.05 mol) in water (50 ml) and stirred vigorously at 20°C for one hour. Sodium cyanide (4.9 g, 0.1 mol) was added and the mixture stirred for a further hour at 20°C. The ether layer was separated, dried ($MgSO_4$) and evaporated to give 1D as a colourless oil.

(e) Trans isomer of 2-carboxy-3-azabicyclo[3.2.0]heptane

The product 1D obtained above was refluxed with 6N hydrochloric acid (50 ml) for 4 hours and evaporated to dryness. The sticky residue was passed down an Amberlite (Trade Mark) resin IRA 400, washed successively with sodium hydroxide solution, water, acetic acid, and water, to give 4.0 g of a white solid, melting point 250-252°C (with decomposition). This compound was identified by NMR as the trans isomer of 2-carboxy-3-azabicyclo[3.2.0]heptane.

| Analysis | C | H | N |
|---|---|---|---|
| Calculated for $C_7H_{11}NO_2$ | 59.6 | 7.8 | 9.9 |
| Found | 59.5 | 7.6 | 9.9 |

(f) Cis isomer of 2-carboxy-3-azabicyclo[3.2.0]heptane

5 g of 1D prepared as in (d) above was refluxed for 16 hours with sodium cyanide and isopropyl alcohol. The mixture was evaporated, the residue dissolved in 6N hydrochloric acid (200 ml) and refluxed for 16 hours. The solution was evaporated to dryness, and a mixture of isomers of the desired acid was produced on an Amberlite (Trade Mark) 400 column. The resultant mixture was treated with ethanol and the resultant white solid was identified as the trans isomer of the desired acid. The filtrate was evaporated, and treated with acetone to give 1.8 g of white crystals, which were shown by NMR to be a mixture of cis and trans isomers.

Evaporation of the acetone solution gave a brown residue which on treating with a little acetone gave 1.0 g of a brown

solid, melting point 217-220°C, which was shown by NMR to be a mixture of cis isomer with a little trans isomer.

The brown solid was dissolved in 1.5 N hydrochloric acid and purified by chromatography on an Amberlite (Trade Mark) resin followed by regeneration of the free acid as 400 mg white crystals, melting point 215-216°C. This substance was identified by NMR as the cis isomer of 2-carboxy-3-azabicyclo[3.2.0]heptane.

| Analysis | C | H | N |
|---|---|---|---|
| Calculated for $C_7H_{11}NO_2$ | 59.6 | 7.8 | 9.9 |
| Found | 57.2 | 7.9 | 9.5 |

Example 2 - Preparation of 2-ethoxycarbonyl-3-azabicyclo[3.2.0] heptane

2-carboxy-3-azabicyclo[3.2.0]heptane (1.41 g, 0.01 mol) was dissolved in ethanol (50 ml) and thionyl chloride (20 ml) was added slowly. The mixture was refluxed for 8 hours and evaporated. The residue was treated with sodium bicarbonate solution and extracted with ethyl acetate (2 x 500 ml). The ethyl acetate layer was separated, dried ($Na_2SO_4$) and evaporated to give a brown oil which was distilled under reduced pressure to give 1 g of the desired product as a colourless oil, boiling point 78-80°C at a pressure of 0.65 mm Hg.

| Analysis | C | H | N |
|---|---|---|---|
| Calculated for $C_9H_{15}NO_2$ | 63.9 | 8.9 | 8.3 |
| Found | 64.0 | 9.3 | 8.2 |

Example 3 - Preparation of the sodium salt of 2-carboxy-3-azabicyclo[3.2.0]heptane

2-carboxy-3-azabicyclo[3.2.0]heptane (0.705 g, 0.005 mol) and sodium hydroxide (0.200 g, 0.005 mol) were dissolved in water (20 ml). The mixture was evaporated to dryness. The residue was treated several times with ethanol and then with isopropyl alcohol to give 0.65 g of a pale yellow solid, melting point 240-240°C (with decomposition). The IR spectrum suggested that the product might be a hydrated salt.

Example 4 - Preparation of 2-carboxy-3,4-dimethylpyrrolidine

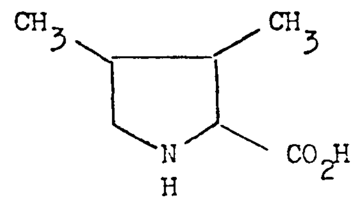

(a) 1-methylcarbonyl-2,2-diethoxycarbonyl-3,4-dimethyl-5-hydroxy-pyrrolidine (4A)

Diethylacetamido malonate (43.2 g, 0.2 mol) and sodium (0.46 g, 0.02 mol) were dissolved in ethanol (200 ml), and tigaldehyde ($CH_3.CH = C(CH_3).CHO$) (18.48 g, 0.22 mol) was added at 5-10°C. The mixture was stirred at 20°C for 3 days and then acidified with glacial acetic acid (4.0 ml). The mixture was evaporated and the residue dissolved in diethyl ether (200 ml) and filtered. On standing, the mixture deposited 18.0 g of 4A as white crystals, melting point 102-104°C.

| Analysis | C | H | N |
|---|---|---|---|
| Calculated for $C_{14}H_{23}NO_6$ | 55.8 | 7.6 | 4.7 |
| Found | 55.8 | 7.6 | 4.5 |

(b) 6.02 g (0.02 mol) of 4A were dissolved in concentrated hydrochloric acid (100 ml) and heated to reflux with gradual addition of excess tin. The mixture was refluxed overnight and then excess tin salts were removed using hydrogen sulphide. The solution was evaporated to dryness and the residue was passed down an Amberlite (trade mark) IRA 400 column using acetic acid, to give 2.0 g of the desired product as a white solid, melting point 218-220°C.

| Analysis | C | H | N |
|---|---|---|---|
| Calculated for $C_7H_{13}NO_2$ | 58.7 | 9.1 | 9.8 |
| Found | 58.7 | 9.0 | 9.4 |

Example 5 - 2-ethoxycarbonyl-3,4-dimethylpyrrolidine

By a method analogous to that described in Example 2, the desired compound was prepared from the compound of Example 4, and ethanol. It was a pale yellow oil.

| Analysis | C | H | N |
|---|---|---|---|
| Calculated for $C_9H_{17}NO_2$ | 63.2 | 9.9 | 8.2 |
| Found | 63.5 | 9.6 | 7.8 |

Example 6 - The hydrazide of 2-carboxy-3,4-dimethylpyrrolidine

0.513 g (0.003 mol) of the compound of Example 4 was refluxed for 16 hours with a five-fold molar excess of hydrazine using ethanol as solvent. The mixture was then evaporated and the residue crystallised from toluene to give 0.3 g of the desired product as white crystals, melting point 99-100°C.

| Analysis | C | H | N |
|---|---|---|---|
| Calculated for $C_7H_{15}N_3O$ | 50.6 | 9.6 | 25.3 |
| Found | 50.6 | 9.8 | 25.2 |

Example 7 - Demonstration of pollen-suppressing activity

Spring wheat, variety Sicco, was propagated in a glasshouse in 13 cm pots containing a loam-based compost. Supplementary lighting was provided by high-pressure mercury vapour lamps to give a constant daylength of 16 hours. The temperature was maintained at approximately 20°C.

The compound to be tested was formulated as an aqueous solution containing 0.1% nonidet P 40 (trade mark) as wetting agent and 1% acetone to aid solubility. This formulation was diluted with water to a concentration of active ingredient of 800 ppm and sprayed onto plants at a volume corresponding to 1000 litres/ha. The plants were treated at the growth stage when the second node of the plant was just detectable.

At ear emergence but before anthesis, 5 heads from each treated pot were placed in cellophane bags to prevent cross-pollination. At maturity, the bagged ears were harvested, and seed set was recorded and compared with untreated controls.

The results obtained using various pyrrolidine derivatives are shown in the following Table. These results clearly illustrate the ability of the compounds to sterilise the male anthers of wheat.

TABLE

| Compound Prepared in Example No. | Reduction in seed set % of control |
|---|---|
| 1e | 23 |
| 1f | 16 |
| 3 | 33 |
| 4 | 16 |
| 5 | 39 |
| 6 | 10 |

CLAIMS

1. A method of sterilising the male anthers of a plant, which comprises applying to the plant a pyrrolidine derivative which is a compound of the general formula I or a salt and/or an ester, N-alkylamide, N,N-dialkylamide, the amide or hydrazide thereof:

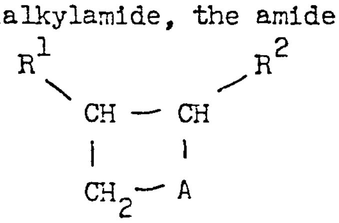

(I)

in which A represents either $-NH-CH-CO_2H$ or $-N=C-CO_2H$, and each of $R^1$ and $R^2$, which may be the same or different represents a halogen atom, a hydroxy group or an alkyl or alkoxy group having from 1 to 6 carbon atoms and optionally substituted by one or more of the same or different halogen atoms, or $R^1$ and $R^2$ together represent an epoxy group, a group of the formula $-O-CH_2-O-$, or a di- or tri-methylene group.

2. A method as claimed in claim 1, in which each of $R^1$ and $R^2$, which may be the same or different, represents an alkyl or alkoxy group having 1 or 2 carbon atoms or a chlorine or bromine atom, or $R^1$ and $R^2$ together represent an epoxy group or a di- or tri-methylene group.

3. A method as claimed in claim 2, in which each of $R^1$ and $R^2$, which may be the same or different, represents a methyl or methoxy group or a chlorine atom, or $R^1$ and $R^2$ together represent a dimethylene group.

4. A method as claimed in any one of claims 1 to 3, in which the pyrrolidine derivative is the free acid of the general formula I, a hydrohalide or an alkali metal salt thereof, the amide or

hydrazide thereof, an alkyl ester thereof having from 1 to 10 carbon atoms in the alkyl group, or a hydrohalide of said ester, amide or hydrazide.

5. A method as claimed in any one of claims 1 to 4, in which at least some of the pyrrolidine derivative is present in its 3,4-cis isomeric form having the structure

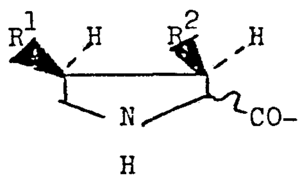 (Ia) or 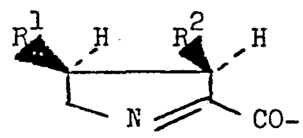 (Ib)

6. A method as claimed in any one of claims 1 to 5, in which A represents $-NH-\overset{|}{C}H-CO_2H$.

7. A method as claimed in claim 1, in which the pyrrolidine derivative is 2-carboxy-3-azabicyclo[3.2.0]heptane or the sodium salt or ethyl ester thereof, or 2-carboxy-3,4-dimethyl-pyrrolidine or the ethyl ester or hydrazide thereof.

8. A method of producing $F_1$ hybrid seed, which includes cross-pollinating a plant which has been treated by a method as claimed in any one of claims 1 to 7, with a second plant of a different strain.

9. A pollen-suppressing composition which comprises a pyrrolidine derivative as defined in any one of claims 1 to 7, together with a suitable carrier.

10. A composition as claimed in claim 9, which comprises at least two carriers, at least one of which is a surface-active agent.

11. A compound of the general formuala I given in claim 1, or a salt and/or an ester, N-alkylamide, N,N-dialkylamide, the amide or hydrazide thereof, in which $R^1$ and $R^2$ have the meanings given in claim 1, with the proviso that, if the compound is the free acid of the general formula I in which A represents $-NH-CH-CO_2H$, and $R^1$ and $R^2$ both represent methyl groups, then these methyl groups are in cis relationship to each other.

12. A compound of the general formula I given in claim 1, in which $R^1$ and $R^2$ have the meanings given in claim 1, with the

proviso that if the compound is a free acid of the general formula I in which A represents $-NH-CH-CO_2H$, then at least one of $R^1$ and $R^2$ is other than a methyl group.

13. A compound as claimed in claim 11, in which each of $R^1$ and $R^2$, which may be the same or different, represents an alkyl or alkoxy group having 1 or 2 carbon atoms or a chlorine or bromine atom, or $R^1$ and $R^2$ together represents an epoxy group or a di- or trimethylene group.

14. A compound as claimed in claim 13, in which each of $R^1$ and $R^2$, which may be the same or different, represents a methyl or methoxy group or a chlorine atom, or $R^1$ and $R^2$ together represent a dimethylene group.

15. A compound as claimed in any one of claims 11 to 14, which is the free acid of the general formula I, a hydrohalide or an alkali metal salt thereof, the amide or hydrazide thereof, an alkyl ester thereof having from 1 to 10 carbon atoms in the alkyl group, or a hydrohalide of said ester, amide or hydrazide.

16. A compound as claimed in any one of claims 11 to 15, in its 3,4-cis isomeric form having the structure

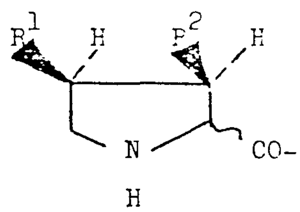 (Ia) or 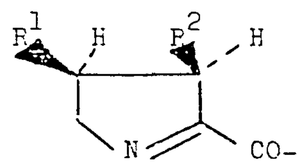 (Ib)

17. A compound as claimed in any one of claims 11 to 16, in which A represents $-NH-CH-CO_2H$.

18. 2-carboxy-3-azabicyclo[3.2.0]heptane or the sodium salt or ethyl ester thereof, or the ethyl ester or hydrazide of 2-carboxy-3,4-dimethylpyrrolidine.

BAD ORIGINAL